Europäisches Patentamt

European Patent Office

Office européen des brevets

(11) Publication number: **0 427 904 A1**

## EUROPEAN PATENT APPLICATION

(21) Application number: 89850401.4

(22) Date of filing: 16.11.89

(51) Int. Cl.5: **C07C 209/88**, C07C 211/27

(43) Date of publication of application:
**22.05.91 Bulletin 91/21**

(84) Designated Contracting States:
**AT BE CH DE ES FR GB GR IT LI LU NL SE**

(71) Applicant: KabiVitrum AB
**Lindhagensgatan 133**
**S-112 87 Stockholm(SE)**

(72) Inventor: **Jönsson, Nils Ake**
**Fotbollsvägen 6**
**S-151 59 Södertälje(SE)**

(74) Representative: **Kummelsten, Per-Arne**
**UPPSALA PATENTBYRA P.O. Box 9013**
**S-750 09 Uppsala(SE)**

(54) A process for preparing R(+)-terodiline and salts thereof.

(57) A novel process for preparing the R(+)-enantiomer of terodiline (N-tert.butyl-1-methyl-3,3-diphenylpropylamine) and pharmaceutically acceptable salts thereof by optical resolution of a racemic mixture of terodiline using tartaric acid, comprises the steps of

a) removing a major part of the S(-)-terodiline enantiomer from the racemic mixture of terodiline by precipitation with L(+)-tartaric acid in an organic solvent,

b) recovering from the mother liquid of step a) a terodiline product which is enriched in the (+)-enantiomer,

c) forming the hydrochloride of said terodiline product in an aqueous solution so as to precipitate R(+)-terodiline hydrochloride from said aqueous solution.

The R(+)-terodiline hydrochloride obtained in step c) is preferably

d) subjected to one or more re-crystallizations from water.

The R(+)-terodiline hydrochloride obtained from step c) or d) may, if desired,

e) be converted into the free base or into a different pharmaceutically acceptable salt by means of conventional procedures.

EP 0 427 904 A1

Xerox Copy Centre

## A PROCESS FOR PREPARING R(+)-TERODILINE AND SALTS THEREOF

### Technical field

The present invention relates to optical resolution of racemic mixtures into the individual enantiomers. More specifically, the invention relates to a novel process for preparing optically pure R(+)-terodiline and salts thereof from a racemic mixture of terodiline [(+/-)-N-tert.-butyl-1-methyl-3,3-diphenylpropylamine].

### Background of the invention

Terodiline is an antichlorinergic substance which in the form of its hydrochloride (terodiline hydrochloride) is widely used to treat urinary incontinence. It is known that the anticholinergic activity mainly resides in the R(+)-form of the compound, and that the R(+)- and S(-)-forms are differently metabolized (B. Lindeke, Ö. Ericsson, Å. Jönsson and B. Vangbo. Xenobiotica 17 (1987) 1269). It is thus desirable to use the pure R(+) enantiomer in therapy.

A process for the resolution of (+/-)-terodiline using (+)- and (-)-tartaric acid has been described (loc. cit.). However, this process is uneconomic on an industrial scale as it requires the use of the very expensive D(-)- form of tartaric acid, which is used to form a crystalline salt with R(+)-terodiline. There is thus a need of improved processes for preparing optically pure R(+)-terodiline hydrochlorides.

### Object of the invention

The primary object of the invention is to provide a novel process for recovering optically pure R(+)-terodiline and pharmaceutically acceptable salts thereof from racemic terodiline.

A special object of the invention is to provide a process of the indicated type, which does not require expensive reactants, is simple and convenient to carry out and produces an end product of high optical purity in high yields.

These and other objects of the invention will be explained further in the following description of the invention.

### Summary of the invention

The above and related objects of the invention have been achieved by means of a novel process for preparing the R(+)-enantiomer of terodiline (N-tert.butyl-1-methyl-3,3-diphenylpropylamine) and pharmaceutically acceptable salts thereof by optical resolution of a racemic mixture of terodiline using tartaric acid, which comprises the steps of

a) removing a major part of the S(-)-terodiline enantiomer from the racemic mixture of terodiline by precipitation with L(+)-tartaric acid in an organic solvent,

b) recovering from the mother liquid of step a) a terodiline product which is enriched in the (+)-enantiomer,

c) forming the hydrochloride of said terodiline product in an aqueous solution so as to precipitate R(+)-terodiline hydrochloride from said aqueous solution.

The R(+)-terodiline hydrocloride obtained in step c) is preferably

d) subjected to one or more re-crystallizations from water.

If desired, the R(+)-terodiline hydrochloride obtained from step c) or d) may

e) be converted into the free base or into a different pharmaceutically acceptable salt by means of conventional procedures.

In step a) it is preferred to use from about 0.8 to about 1.5 moles of (+)-tartaric acid per 1 mole of the terodiline racemate, most preferably approximately equimolar amounts. Other preferred embodiments of the claimed process are disclosed below and/or are indicated in the sub-claims.

By means of this novel process it has become possible to circumvent the use of expensive D(-)-tartaric acid in the resolution by taking advantage of the differences in solubility in water between the terodiline hydrochloride in the (+)-form and that of the racemate of terodiline. In particular, it has surprisingly been found that the R(+)-form of terodiline and its pharmaceuticallyacceptable salts can be obtained in very high purity and high yields despite the fact that the differences in water solubility are not very large (the solubility in water at 10°C is 24 g/L for the (+/-)-form and 14 g/L for the (+)-form). In particular, the invention makes it possible to design a simple and economic process for the production of R(+)-terodiline hydrochloride from the racemic amine in a yield of at least 60% and an optical purity higher than 99%.

### Description of preferred embodiments

In a first preferred embodiment of the process of the process according to the invention, most of the S(-)-terodiline is first removed from a solution of the racemate in a suitable solvent, preferably ethanol, by precipitation with an approximately equimolar amount of L(+)-tartaric acid, whereupon amine enriched in the (+)-form is recovered from the mother liquor and the (+)-hydrochloride is precipitated from water with hydrochloric acid.

An alternative preferred embodiment comprises the same initial step as the first embodiment, viz. precipitation of S(-)-terodiline hydrogen L(+)-tartrate. The mother liquor from this precipitation step is then concentrated to a thick syrup and worked up without isolation of the free amine. The syrup is dissolved in water whereupon the solution is adjusted to pH about 5 to 6 using a water soluble base, preferably ammonia, that forms readily water soluble salts with L(+)-tartaric acid. R(+)-terodiline hydrochloride is then precipitated from the solution by the addition of a readily water soluble chloride salt that forms a readily water soluble salt with L-(+)-tartaric acid, the preferred salt being ammonium chloride.

The crude (+)-terodiline hydrochloride thus obtained is preferably further purified by recrystallization from water. One or two such crystallizations produce R(+)-terodiline hydrochloride of better than 99% enantiomeric purity and a melting point of 224-225°C. (The racemic material occurs in several polymorphic forms, the highest melting of which melts at 186-188°C). If desired, the obtained hydrochloride can be converted into the free amine or to other pharmaceutically acceptable salts in conventional manner.

The following working examples are illustrations of how the process according to the invention can be put into prac tice.

### EXAMPLE 1

(+/-)-Terodiline (2.50 kg; 8.88 mol) and L(+)-tartaric acid (1.34 kg; 8.93 mol) are dissolved in 10 L of 95% ethanol at 40-50°C. The clear solution is chilled to about 35°C and seeded with about 1 g of pre-prepared S(-)-terodiline hydrogen L(+)-tartrate. After cooling at about 10-20°C for a few hours the crude salt is sucked off, washed with about 5 L of ethanol and recrystallized from 8 L of boiling ethanol with cooling as above. The crystalline material is sucked off and washed with about 3 L of ethanol and dried to yield 1.95 kg of S(-)-terodiline hydrogen L(+)-tartrate.

The combined mother liquors and washings from the above procedures are evaporated under reduced pressure at 50-100°C to a thick syrup.

This is dissolved in about 10 L of hot water and the solution is made strongly alkaline with 45% aqueous sodium hydroxide solution. The free amine separates as an upper layer which is collected and washed once with water. It is suspended in about 8 L of water at about 50°C and neutralized to about pH 4-5 with concentrated hydrochloric acid (about 380 ml). The solution is then chilled to about 10°C for several hours. The precipated salt is sucked off and washed with cold water. A dried sample melts at 215-220°C. The product is recrystallized from 4 L of boiling water with cooling and isolation as above. After drying 0.90 kg (64% of theoretical) of R(+)-terodiline hydrochloride is obtained with m.p. 224-225°C (Kofler bench) and an enantiomeric purity of >99%, determined using HPLC on a chiral AGP column.

### EXAMPLE 2

S(-)-Terodiline hydrogen L(+)-tartrate is isolated from 844 g (3.00 mol) of (+/-)-terodiline using 450 g (3.00 mol) of L(+)-tartaric acid in 4.5 L of 95% ethanol following the procedure of Example 1. The combined mother liquors and washings are concentrated to a thick syrup at 50-100°C under vacuum. The syrup is dissolved in 6 L of boiling water. Remaining alcohol is removed by distilling off about 1 L of solvent. The volume is restored by the addition of water and ammonia is added to pH 5-6. Solid ammonium chloride (120 g) is then added and the solution is allowed to cool to room temperature.

The precipitated crude R(+)-terodiline hydrochloride is sucked off and washed with cold water. The moist material (a dried sample melts at about 218-220°C) is recrystallized from 4 and 2 L of water to yield 310 g (65% of theoretical) of pure R-(+)-terodiline hydrochloride melting at 224-225°C (Kofler bench) and having an enantiomeric purity, determined as in Example 1, of better than 99%.

### Claims

1. A process for preparing the R(+)-enantiomer of terodiline (N-tert.butyl-1-methyl-3,3-diphenylpropylamine) and pharmaceutically acceptable salts thereof by optical resolution of a racemic mixture of terodiline using tartaric acid, comprising the steps of

    a) removing a major part of the S(-)-terodiline enantiomer from the racemic mixture of terodiline by precipitation with L(+)-tartaric acid in an organic solvent,

    b) recovering from the mother liquid of step a) a terodiline product which is enriched in the (+)-enantiomer,

c) forming the hydrochloride of said terodiline product in an aqueous solution so as to precipitate R(+)-terodiline hydrochloride from said aqueous solution,

d) optionally re-crystallizing the precipitated R-(+)-terodiline hydrochloride from water, and

e) if desired, converting the R(+)-terodiline hydrochloride into the free base or into a different pharmaceutically acceptable salt.

2. A process according to claim 1, wherein step c) comprises treatment of said terodiline product with hydrochloric acid in water.

3. A process according to claim 1 or 2, which further comprises isolation of the free amine before step c).

4. A process according to claim 1, wherein step c) comprises treatment in water of said terodiline product with a water soluble base, which readily forms water soluble salts with L(+)-tartaric acid, and precipitation of R(+)-terodiline hydrochloride from the solution by addition of a chloride salt of a base which forms a readily water soluble salt with L(+)-tartaric acid.

5. A process according to claim 4, wherein said treatment with a water soluble base comprises adjustment of the pH of the aqueous solution to about 5 to 6.

6. A process according to claim 4 or 5, wherein said water soluble base is ammonia.

7. A process according to claim 4, 5 or 6, wherein said chloride salt is ammonium chloride.

8. A process according to any one of the preceding claims, wherein the precipitation of the S(-)-terodiline enantiomer in step a) includes seeding with S(-)-terodiline hydrogen L(+)-tartrate.

9. A process according to any one of the preceding claims, wherein the precipitation of the S(-)-terodiline enantiomer in step a) comprises combining racemic terodiline with an approximately equimolar amount of L(+)-tartaric acid in an organic solvent.

10. A process according to claim 9, wherein said organic solvent is a lower alkanol, especially ethanol.

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| D,X | XENOBIOTICA, vol. 17, no. 11, 1987, pages 1269-1278; B. LINDEKE et al.: "Biotransformation of terodiline. III. Opposed stereoselectivity in the benzylic and aromatic hydroxylations in rat liver microsomes" <br> * Page 1270, last paragraph - page 1271, line 19 * | 1-10 | C 07 C 209/88 <br> C 07 C 211/27 |
|  |  |  | TECHNICAL FIELDS SEARCHED (Int. Cl.5) |
|  |  |  | C 07 C 209/00 <br> C 07 C 211/00 |

The present search report has been drawn up for all claims

| Place of search | Date of completion of the search | Examiner |
|---|---|---|
| THE HAGUE | 02-07-1990 | HELPS I.M. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)